# FASCICULE DE BREVET EUROPEEN

(11) **EP 0 720 425 B1**
(45) Date de publication et mention de la délivrance du brevet: **25.02.1998**
(21) Numéro de dépôt: 94928425.1
(22) Date de dépôt: 21.09.1994
(51) Int. Cl.: A01H 4/00

(54) **PROCEDE POUR FAVORISER L'EMBRYOGENESE SOMATIQUE SECONDAIRE ET APPLICATION A LA REGENERATION DE PLANTES, EN PARTICULIER DE LA VIGNE**
VERFAHREN ZUR FÖRDERUNG DER SEKUNDÄREN SOMATISCHEN EMBRYOGENESE UND ANWENDUNG ZUR PFLANZENREGENERATION, INSBESONDERE DER WEINREBE
METHOD FOR ENCOURAGING SECONDARY SOMATIC EMBRYOGENESIS AND APPLICATION TO THE REGENERATION OF PLANTS, IN PARTICULAR THE GRAPE VINE

(30) Priorité: 22.09.1993 FR 9311287
(43) Date de publication de la demande: 10.07.1996
(73) Titulaire: L.V.M.H. RECHERCHE, F-92000 Nanterre (FR)
(72) Inventeur: MAES, Olivier, Saskatoon, Saskatchewan S7N 2S2 (CA); JOUENNE, Thierry, F-76000 Rouen (FR); GUERN, Jean, F-91190 Gif-sur-Yvette (FR); COUTOS-THEVENOT, Pierre, F-75005 Paris (FR)
(74) Mandataire: Ahner, Francis
(86) Numéro de dépôt international: FR9401105
(87) Numéro de publication internationale: WO9508262

(56) Documents cités:
- EP-A- 0 293 598
- WO-A-87/02701
- US-A- 4 532 733
- PLANT CELL, TISSUE AND ORGAN CULTURE, vol.9, no.1, 1987, DORDRECHT, NL pages 73 - 80 D.J. GRAY ET AL. 'Initiation and maintenance of long term somatic embryogenesis from anthers and ovaries of Vitis longii '
- IN VITRO, vol.29A, no.3, Mars 1993 page 63A L.C. MARTINELLI ET AL. 'A study for the characterization of somatic embryogenesis on grapevine'
- PLANT SCIENCE, vol.86, 1992 pages 137 - 145 P. COUTOS-THEVENOT ET AL. 'Extracellular protein patterns of grapevine cell suspensions in embryogenic and non-embryogenic situations'
- ANNALS OF BOTANY, vol.57, 1986 pages 109 - 117 G. MAHESWARAN ET AL. 'Direct secondary somatic embryogenesis from immature sexual embryos of Trifolium repens cultured in vitro'
- THE NEW PHYTOLOGIST, vol.95, 1983 pages 349 - 358 C.-S- LOH ET AL. 'Cytokinins and the regeneration of plantlets from secondary embryoids of winter oilseed rape, Brassica napus ssp. oleifera'
- PLANT CELL, TISSUE AND ORGAN CULTURE, vol.29, 1992 pages 125 - 133 P. COUTOS-THEVENOT ET AL. 'Somatic embryogenesis from grapevine cell. I - Improvement of embryo development by changes in culture conditions'

## Description

La présente invention se rapporte à un procédé permettant de favoriser l'embryogénèse secondaire à partir d'embryons somatiques primaires en culture et à l'application de ce procédé à la regénération de plantes en particulier de la vigne.

Pour la majeure partie des espèces végétales, la production d'embryons in natura se fait par voie sexuée ce qui a l'inconvénient, pour les espèces hétérozygotes telles que la vigne par exemple, d'entraîner une recombinaison des caractères génétiques et de perdre ainsi les caractéristiques principales des parents dont ils sont issus. Dans le cas de la vigne, les caractéristiques du cépage seraient perdues, ce qui est inacceptable en oenologie.

Pour permettre une multiplication clonale, c'est-à-dire à l'identique, plusieurs méthodes ont été développées, notamment in vitro. Elles peuvent aboutir à la formation d'embryons semblables à ceux issus de la fécondation des gamètes mâles et femelles, mais qui sont au niveau génétique une copie de la plante dont ils sont issus. Ces embryons sont appelés somatiques, leur production suit en général le schéma suivant :
1) induction du potentiel embryogène par des auxines exogènes, généralement en fortes concentrations, qui permettent l'apparition d'agrégats (ou masses) proembryogènes (PEM) qui correspondent à des groupes de 10 à 50 cellules, notamment méristèmatiques. Il arrive aussi que les embryons soient obtenus directement sur un tel milieu.
2) transfert de ces cellules sur des milieux exempts d'auxines voire à faible concentration en auxine, qui conduisent à la formation d'embryons somatiques à partir de ces PEM, en suivant les différents stades d'évolution de l'embryogénèse végétale. Ces stades d'évolution sont généralement caractérisés par une forme particulière. Ainsi dans le cas de la vigne, il est habituel de distinguer successivement les stades suivants : globule, coeur, torpille et plantule.

Ces principes sont décrits, en ce qui concerne la propagation de cellules de vigne, dans la demande de brevet US 4 532 733.

Par ailleurs, il est souhaitable dans certains cas pour obtenir des plantes transgéniques, d'utiliser des embryons somatiques comme source de matériel végétal de transformation. En utilisant dans ce cas les techniques de transformation classiquement utilisées, telles que la coculture avec des agro-bactéries, ou la biolistique, on obtient des embryons dont certaines zones seulement sont transformées. La possibilité de favoriser l'embryogénèse secondaire, ou encore appelée embryogénèse adventive, à partir des zones transformées, et donc d'augmenter l'efficacité du système transformation-regénération, présente donc un intérêt évident. D'autre part, dans le cas où la transformation génétique des embryons doit être effectuée, favoriser la formation d'embryons secondaires, au niveau de l'épiderme des embryons transformés, au lieu d'un développement normal des pousses est aussi très important pour augmenter le nombre de transformants produits.

C'est pourquoi la présente invention a pour objet un procédé pour favoriser la production d'embryons végétaux secondaires à partir d'une culture d'embryons somatiques primaires, caractérisé en ce qu'on introduit dans le milieu de culture desdits embryons somatiques primaires une protéine inhibitrice de l'embryogénèse primaire en concentration efficace pour obtenir l'embryogénèse secondaire.

On appelle inhibition de l'embryogénèse primaire l'arrêt du développement à un stade de l'évolution de l'embryogénèse qui peut être le stade globule ou coeur ou torpille.

Plus particulièrement, le procédé selon la présente invention peut s'appliquer à la production d'embryons somatiques de vigne.

De manière générale, dans le cas des suspensions de cellules embryogènes du type 41 B, en culture dans des conditions classiques sans transfert fréquent, on observe un blocage rapide du développement au stade globule ou coeur. Certains auteurs ont avancé que ce phénomène d'inhibition était dû à la présence de macromolécules extra-cellulaires supérieures à 10 kDa (Plant Cell Tiss. Org. Cult. (1992) 29, 125-133).

La Demanderesse a trouvé de manière surprenante que certaines protéines et notamment une certaine classe de celles réputées pour inhiber l'embryogénèse primaire, permettaient de favoriser l'embryogénèse secondaire.

Plus particulièrement, le procédé selon l'invention peut être mis en oeuvre en introduisant dans le milieu du culture des embryons primaires une fraction contenant au moins une protéine, extraite d'une culture d'embryons végétaux bloqués dans leur développement.

Selon un mode particulier de réalisation de la présente invention,
1) on fait une culture d'embryon de la plante considérée,
2) on isole les protéines inhibitrices de l'embryogénèse, et
3) on introduit ces protéines isolées dans la culture d'embryon dont on veut obtenir des embryons secondaires.

De préférence la culture dont on extrait la fraction précitée est une culture âgée d'environ 15 jours, et dont la densité initiale était élevée, par exemple de l'ordre de 1 à 2 µl de volume d'agrégats cellulaires sédimentés (PCV) par millilitre de milieu.

Selon une variante particulière, la protéine est obtenue à partir d'une culture d'embryons végétaux appartenant à la même espèce que lesdits embryons somatiques primaires.

De préférence, la protéine est une protéine extracellulaire extraite d'un milieu de culture d'embryons végétaux.

Parmi les protéines extracellulaires convenant pour mettre en oeuvre le procédé selon l'invention, il est avantageux de choisir les protéines anioniques, éventuellement glycosylées, présentant un poids moléculaire apparent compris entre 32 kDa environ et 66 kDa environ en SDS-PAGE

Une efficacité optimale est obtenue quand le procédé de l'invention est appliqué à des cultures effectuées, au moins partiellement, en l'absence de lumière.

De préférence, la protéine extracellulaire est introduite dans le milieu d'induction et/ou de développement des embryons.

Les embryons primaires selon une variante avantageuse sont au moins partiellement constitués de cellules transformées génétiquement, au moyen d'une technique bien connue de l'homme de l'art.

Les embryons secondaires obtenus suivant le procédé selon la présente invention peuvent être utilisés dans les procédés connus de l'homme de métier pour l'obtention de plantes régénérées.

Par exemple, on pourra utiliser le procédé décrit dans le document US 4 532 733, notamment colonne 3, lignes 27 à 44, comprenant le transfert des embryons dans un milieu de culture de type Murashige et Skoog (1962), supplémenté en cytokinine, telle que la benzyladénine.

Pour ces procédés de regénération à partir d'embryons secondaires, on pourra également se reporter à ceux décrits par G-Maheswaran et al, Ann-Bot., 57 (1988) 109-117 ou par C-S-Loh et ai, New Phytol., 95 (1983) 349-358.

Les exemples qui suivent sont destinés à illustrer l'invention sans aucunement en limiter la portée.

### Exemple 1 : Obtention de protéines extracellulaires d'embryogénèse primaire

### Origine de la culture d'embryon primaire

On utilise comme source d'agrégats de cellules embryogènes, la culture de cellules embryogènes de porte-greffe de vigne 41 B, déposée à l'European Collection of Animal Cell Cultures sous le n° PL 92042917 décrite dans la demande internationale PCT déposée le 11 mai 1993 sous le numéro PCT/FR93/00456. Pour initier les cultures d'embryons dans un milieu dépourvu d'auxine, la suspension cellulaire est filtrée successivement sur des tamis de 500 µm et 200 µm, et la fraction retenue sur 200 µm est lavée trois fois dans un milieu de culture dépourvu d'auxine, selon le protocole décrit précédemment. Les densités de population cellulaire au moment de l'inoculation déterminée par le PCV (Packed Cell Volume, unité de volume cellulaire sédimenté à 1 x g) sont comprises entre 0,05 et 1 µl de PCV.mL⁻¹. La correspondance PCV-densité cellulaire est assurée par des numérations faites par la méthode de dispersion dans l'acide chromique.

### Purification des protéines extracellulaires

On réalise une culture d'embryons, comme indiqué ci-dessus, à une densité d'inoculation initiale de 1 µl de PCV par millilitre de milieu. Aucune sous-culture journalière n'est pratiquée. Après 14 jours de culture, alors que les embryons sont bloqués dans leur développement au stade globule ou coeur, comme décrit par P. Coutos-Thevenot et al. (Plant Science, (1992) 86 137-145), on extrait les protéines extracellulaires sécrétées pendant l'embryogénèse somatique. Le milieu de cette culture est clarifié par filtration sous vide à travers des filtres de particules de verre (Pyrex France) puis des membranes de fibres de verre (GF/C, Whatman) avant d'être concentré de 100 à 200 fois, en utilisant le système d'ultrafiltration Amicon équipé d'une membrane ayant un seuil de coupure de 3000 Daltons (YM 3). Le concentré est appliqué à une colonne CLHP d'échange de cations (SP-5PW, Waters) équilibrée avec un tampon phosphate (Na₂HPO₄-NaH₂PO₄, 25 mM, pH 6,5) à un débit de 0,8 mL.min⁻¹. On recueille la fraction des protéines non retenues, détectée par densité optique et suivie à 280 nm sur un détecteur UV (M440, Waters). Cette fraction de protéine non retenues (70% des protéines initiales) est réinjectée sur une colonne d'échange d'anions (DEAE-5PW, Waters) équilibrée avec un tampon TRIS (TRIS-HCl, 25 mM, pH 8,5) à 0,8 mL.min⁻¹. La colonne est ensuite éluée en 40 minutes par un gradient linéaire de solution saline (TRIS-HCl 25 mM, NaCl 1 M, pH 8,5) de 0 à 0,3 M. Différents pics d'élution sont observés et sont recueillis dans des fractions séparées. Ces fractions sont conservées à 4° C. Les fractions protéiques sont stérilisées par filtration à 0,22 µm et leur concentration déterminée par la méthode de Lowry en utilisant de la sérum albumine bovine comme étalon. Les fractions protéiques receuillies pendant l'élution de la colonne anionique (DEAE) pendant les 25 premières minutes du gradient constituent un pic complexe avec trois épaulements pour les fractions 1 à 3, alors que les autres fractions sont recueillies sous forme de pics séparés. Au total, 8 fractions ont été recueillies.

Pour caractériser les protéines présentes dans chaque fraction, en estimant leur masse molaire, on pratique une électrophorèse de type SDS-PAGE à voltage constant (200 V). Le gel est ensuite coloré au bleu de Coomassie ou révélé par la technique dite de Western blot avec le complexe Concanavaline A-péroxidase (Bio-rad Laboratoires, Richmond CA).

Pour chaque fraction, on observe plusieurs peptides. Il apparait que toutes ces fractions contiennent au moins une protéine glycosylée, comme déterminé par Western blot. Les trois premières fractions apparaissent comme des mélanges complexes de peptides dans une large gamme de masses moléculaires, alors que les fractions 4 à 7 apparaissent moins complexes et composées de polypeptides différents, allant de 32 à 66 kDa.

### Exemple 2 : Production d'embryogénèse secondaire par complémentation en protéines inhibitrices de l'embryogénèse primaire

Les masses proembryogènes inoculées à 0,1 µl de PCV.mL⁻¹ sont complémentées au début de la culture dans un milieu exempt d'auxine avec l'une des fractions protéiques recueillies suivant l'exemple 1. Les expériences sont réalisées pour chacune des fractions dans des boites de microtitration de 24 puits dans 1 mL de milieu liquide et cultivées à l'obscurité sur un agitateur orbital (130 rpm) à 26° C. Pour chaque culture on effectue quatre répétitions indépendantes. Parallèlement des cultures de masse proembryogènes témoins sont réalisées dans les mêmes conditions, si ce n'est qu'au lieu d'ajouter une fraction protéique, on ajoute un volume identique de tampon CLHP, correspondant à une concentration en NaCl 0,25 M, qui est supérieure à celle nécessaire pour éluer les fractions protéiques de la colonne.

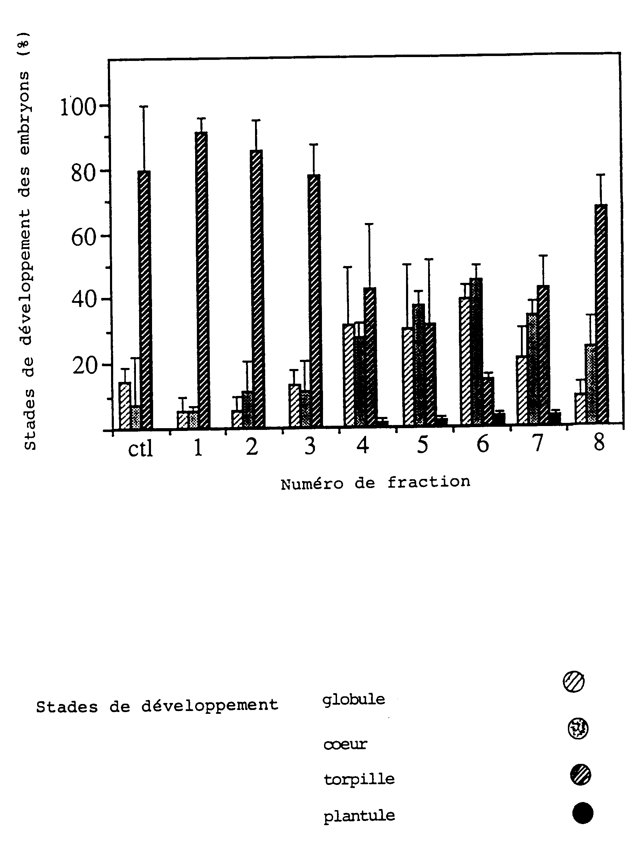

Dans des expériences préliminaires d'inhibition protéique, les fractions protéiques sont ajoutées à des concentrations allant de 1 à 100 fois leur concentration initiale trouvée dans les cultures inoculées à haute densité cellulaire et le I₅₀ (dose inhibant le développement de 50% des embryons) pour chaque fraction varie de 10 à 15 µg.mL⁻¹. Les fractions protéiques sont ensuite toutes testées à 15 µg.mL⁻¹ et leur influence sur la progression embryogène est mesurée après 20 jours de culture. Les embryons de chaque puits sont dénombrés. Les stades de développement pris en considération sont les stades globule, coeur, torpille et plantule. Les résultats sont rassemblés sur la figure unique annexée.

Sur la figure unique annexée, on a représenté, pour chaque fraction protéique testée et pour les cultures témoins, le nombre d'embryons pour chaque stade de développement pris en considération, exprimé en pourcentage par rapport au nombre total d'embryons dénombrés dans chaque puits.

On observe, en ce qui concerne le témoin, que 80% des embryons se sont développés jusqu'au stade torpille. Il en est environ de même pour les cultures traitées par les fractions 1, 2, 3 et 8, pour lesquelles 70 à 90% des embryons sont parvenus également au stade torpille.

Par contre, les résultats obtenus avec les fractions 4, 5, 6 et 7 qui contiennent des protéines de masse molaire de 32 kDa à 66 kDa, sont très sensiblement différents.

Pour les fractions 4 et 5, on trouve une proportion sensiblement identique, de l'ordre de 30 ou 4O% d'embryons à chacun des trois stades globule, coeur et torpille. Pour la fraction 6, les embryons aux stades globule et coeur sont prépondérants. Tandis que pour la fraction 7, le nombre d'embryons parvenus au stade torpille est supérieur à celui des embryons moins développés. Toutefois, pour cette fraction, la proportion d'embryons bloqués au premier stade de développement, c'est-à-dire au stade globule est supérieure à 20 %, ce qui est très sensiblement supérieur à la proportion des embryons du même stade dénombrés pour les fractions 1, 2, 3 et 8 et pour les cultures témoins. Ainsi, il apparait clairement que le traitement des cultures d'embryons par les fractions 4, 5, 6 et 7, entraîne une diminution très notable du nombre d'embryons développés au stade torpille et une plus forte proportion d'embryons au stade globule. On peut donc en conclure que ces fractions ont provoqué l'inhibition du développement des embryons somatiques.

On remarquera toutefois que, bien que le développement des embryons soit bloqué pour la majorité d'entre eux, une faible proportion a échappé à cette inhibition et pour laquelle les embryons ont atteint le stade de plantule.

Une observation microscopique de ces plantules recueillies montre que, sur l'épiderme de celles-ci, se sont formé de nombreux embryons secondaires, ou adventifs, dont le développement semble s'être arrêté au stade globulaire.

Ainsi, la complémentation de culture d'embryons somatiques primaires, avec des fractions extraites de cultures d'embryons dont le développement était bloqué, a permis d'obtenir la formation d'une grande quantité d'embryons secondaires, qu'il sera possible de développer ultérieurement, après transplantation dans un milieu approprié, suivant l'un des procédés bien connus de l'homme de l'art, comme décrit plus haut, pour obtenir la regénération de nouvelles plantes.

## Revendications

1. Procédé pour favoriser la production d'embryons végétaux secondaires à partir d'une culture d'embryons somatiques primaires, caractérisé en ce qu'on introduit dans le milieu de culture desdits embryons somatiques primaires une protéine inhibitrice de l'embryogénèse primaire en concentration efficace pour obtenir l'embryogénèse secondaire.

2. Procédé pour favoriser la production d'embryons végétaux secondaires à partir d'une culture d'embryons somatiques primaires selon la revendication 1, caractérisé en ce que la protéine inhibitrice de l'embryogénèse primaire est introduite sous forme d'une fraction contenant au moins une protéine, extraite d'une culture d'embryons végétaux bloqués dans leur développement.

3. Procédé selon la revendication 2, caractérisé en ce que la culture dont on extrait la fraction précitée est une culture âgée d'environ 15 jours, et dont la densité initiale était élevée, par exemple de l'ordre de 1 à 2 µl de volume d'agrégats cellulaires sédimentés (PCV) par millilitre de milieu.

4. Procédé selon l'une des revendications 1 à 3, caractérisé en ce que la protéine précitée est obtenue à partir d'une culture d'embryons végétaux appartenant à la même espèce que lesdits embryons somatiques primaires.

5. Procédé selon l'une des revendications 1 à 4, caractérisé en ce que la protéine précitée est une protéine extracellulaire extraite d'un milieu de culture d'embryons végétaux.

6. Procédé selon l'une des revendications 1 à 5, caractérisé en ce que la protéine précitée est une protéine de type anionique, éventuellement glycosylée.

7. Procédé selon l'une des revendications 1 à 6, caractérisé en ce que la protéine précitée présente un poids moléculaire apparent compris entre 32 kDa et 66 kDa en SDS-PAGE.

8. Procédé selon l'une des revendications 1 à 7, caractérisé en ce que la protéine précitée est introduite dans le milieu d'induction et/ou de développement des embryons.

9. Procédé selon l'une des revendications 1 à 8, caractérisé en ce que les embryons somatiques primaires sont au moins partiellement constitués de cellules génétiquement transformées.

10. Procédé selon l'une des revendications 1 à 9, caractérisé en ce que la culture d'embryons primaires est une culture d'embryons de vignes.

## Claims

1. Method for favouring the production of secondary plant embryos from a culture of primary somatic embryos, characterized in that a protein which inhibits primary embryogenesis is introduced into the culture medium of the said primary somatic embryos at a concentration which is effective for obtaining secondary embryogenesis.

2. Method for favouring the production of secondary plant embryos from a culture of primary somatic embryos according to Claim 1, characterized in that the protein which inhibits primary embryogenesis is introduced in the form of a fraction containing at least one protein which is extracted from a culture of plant embryos which are blocked in their development.

3. Method according to Claim 2, characterized in that the culture from which the abovementioned fraction is extracted is a culture which is approximately 15 days old and which had a high initial density, for example of the order of from 1 to 2 µl of packed cell volume (PCV) per millilitre of medium.

4. Method according to one of Claims 1 to 3, characterized in that the abovementioned protein is obtained from a culture of plant embryos which belong to the same species as the said primary somatic embryos.

5. Method according to one of Claims 1 to 4, characterized in that the abovementioned protein is an extra-cellular protein which is extracted from a culture medium of plant embryos.

6. Method according to one of Claims 1 to 5, characterized in that the abovementioned protein is a protein of anionic type which may possibly be glycosylated.

7. Method according to one of Claims 1 to 6, characterized in that the abovementioned protein exhibits an apparent molecular weight of between 32 kDa and 66 kDa according to SDS-PAGE.

8. Method according to one of Claims 1 to 7, characterized in that the abovementioned protein is introduced into the medium for inducing and/or developing the embryos.

9. Method according to one of Claims 1 to 8, characterized in that the primary somatic embryos at least partially consist of genetically transformed cells.

10. Method according to one of Claims 1 to 9, characterized in that the culture of primary embryos is a culture of grapevine embryos.

## Patentansprüche

1. Verfahren zur Förderung der Bildung von sekundären pflanzlichen Embryonen ausgehend von einer Kultur primärer somatischer Embryonen, dadurch charakterisiert, daß man zum Kulturmedium dieser primären somatischen Embryonen ein die primäre Embryogenese inhibierendes Protein in einer zur Erzielung der sekundären Embryogenese wirksamen Konzentration gibt.

2. Verfahren zur Förderung der Bildung von sekundären pflanzlichen Embryonen ausgehend von einer Kultur von primären somatischen Embryonen nach Anspruch 1, dadurch charakterisiert, daß das die primäre Embryogenese inhibierende Protein in Form einer Fraktion zugegeben wird die mindestens ein Protein enthält, das aus einer Kultur von in ihrer Entwicklung blockierten pflanzlichen Embryonen extrahiert wurde.

3. Verfahren nach Anspruch 2, dadurch charakterisiert, daß die Kultur, aus der man die vorstehend genannte Fraktion extrahiert, eine Kultur im Alter von ungefähr 15 Tagen ist, deren anfängliche Dichte erhöht ist, beispielsweise in einer Größenordnung von 1 bis 2 ml Volumen an sedimentierten zellulären Aggregaten (PCV) pro Milliliter Medium.

4. Verfahren nach einem der Ansprüche 1 bis 3, dadurch charakterisiert, daß das vorstehend genannte Protein aus einer Kultur von pflanzlichen Embryonen gewonnen wird, die zu derselben Spezies gehören wie die genannten primären somatischen Embryonen.

5. Verfahren nach einem der Ansprüche 1 bis 4, dadurch charakterisiert, daß das vorstehend genannte Protein ein extrazelluläres Protein ist, das aus einem Kulturmedium der pflanzlichen Embryonen extrahiert wurde.

6. Verfahren nach einem der Ansprüche 1 bis 5, dadurch charakterisiert, daß das vorstehende Protein ein Protein vom anionischen Typ ist, gegebenenfalls ein glycosyliertes Protein.

7. Verfahren nach einem der Ansprüche 1 bis 6, dadurch charakterisiert, daß das vorstehend genannte Protein ein scheinbares Molekulargewicht zwischen 32 kDa und 66 kDa in der Natriumlaurylsulfat-Polyacrylamid-Gelelektrophorese (SDS-PAGE) aufweist.

8. Verfahren nach einem der Ansprüche 1 bis 7, dadurch charakterisiert, daß das vorstehend genannte Protein zum Induktionsmedium und/oder dem Entwicklungsmedium der Embryonen gegeben wird.

9. Verfahren nach einem der Ansprüche 1 bis 8, dadurch charakterisiert, daß die primären somatischen Embryonen mindestens teilweise aus genetisch veränderten Zellen bestehen.

10. Verfahren nach einem der Ansprüche 1 bis 9, dadurch charakterisiert, daß die Kultur der primären Embryonen eine Kultur von Weinreben-Embryonen ist.
